# EUROPEAN PATENT APPLICATION

(11) **EP 4 772 787 A1**
(43) Date of publication of application: **08.07.2026**
(21) Application number: 25223417.4
(22) Date of filing: 15.12.2025
(51) Int. Cl.: F16M 11/10, A61B 6/00, F16B 7/10, F16M 11/18, F16M 11/30, F16M 13/02

(54) **TELESCOPIC CYLINDER AND X-RAY IMAGING SYSTEM**

(30) Priority: 07.01.2025 CN 202520027432 U
(71) Applicant: GE Precision Healthcare LLC, Waukesha, WI 53188 (US)
(72) Inventor: YAN, Rongquan, Beijing, 100176 (CN); YANG, Jianqiang, Beijing, 100176 (CN); GU, Shaobo, Beijing, 100176 (CN); LI, Yuqing, Beijing, 100176 (CN); SUN, Jinjie, Beijing, 100176 (CN)
(74) Representative: Kilburn & Strode LLP

(57) **Abstract**

Provided is a telescopic cylinder (100) and an X-ray imaging system. The telescopic cylinder includes a plurality of sleeves arranged in a nested configuration and having mutually parallel central axes. Each inner sleeve cooperates with an outer sleeve adjacent thereto to enable relative sliding such that each inner sleeve can extend and collapse relative to that outer sleeve. The telescopic cylinder further includes guide assemblies. Each guide assembly includes rollers and a rail that are configured as a set. The rollers are provided on left and right sidewalls and rear walls of the inner sleeves and the rails are correspondingly provided on left and right sidewalls and rear walls of the outer sleeves. The rollers and the rail in the same guide assembly are provided on an outer wall surface of the inner sleeve and an inner wall surface of the corresponding outer sleeve, respectively, such that the rollers can roll on guide surfaces defined by the rail, thereby guiding movement of the inner sleeve relative to the outer sleeve. The telescopic cylinder has sufficient structural stability and rigidity while allowing smooth extension and collapse, with good operational stability, low susceptibility to failure, easy maintenance, and low space requirements.

## Description

### TECHNICAL FIELD

The present application relates to the field of imaging, and more particularly, to a telescopic cylinder for a suspension apparatus in an X-ray imaging system and an X-ray imaging system including the telescopic cylinder.

### BACKGROUND

In an X-ray imaging system, X-rays from an X-ray generator are directed towards a subject to be imaged to achieve imaging, the subject to be imaged typically being a patient in a medical diagnostic application.

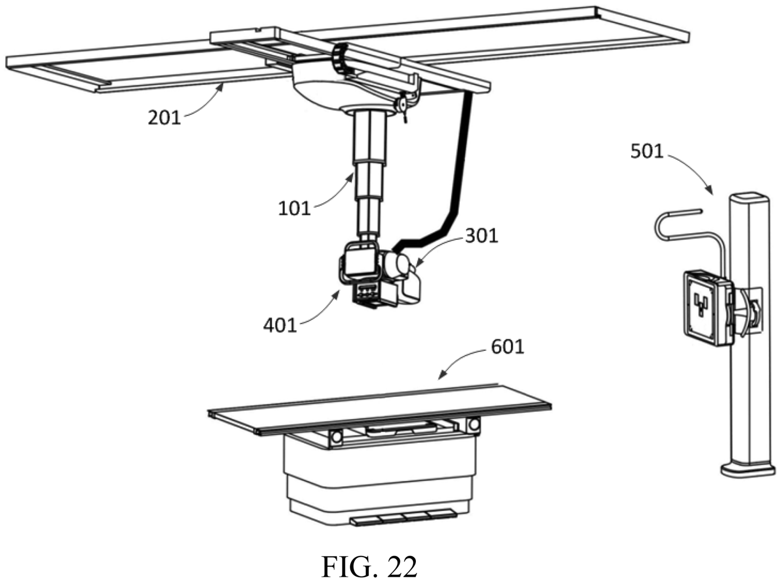

FIG. 22 shows the structure of an X-ray imaging system. As shown in FIG. 22, the X-ray imaging system includes a suspension apparatus (including a telescopic cylinder 101, a main body frame 201, an angle adjustment mechanism 301, and an X-ray generation mechanism 401), a wall stand assembly 501, and an examination table 601. The suspension apparatus may be mounted on a top wall or a wall of a building, etc. The suspension apparatus may include a telescopic cylinder 101, a main body frame 201, an angle adjustment mechanism 301, and an X-ray generation mechanism 401 that are assembled together, such that the suspension apparatus can move within a predetermined range in multiple spatial degrees of freedom, and further the telescopic cylinder 101, the main body frame 201, and the angle adjustment mechanism 301 of the suspension apparatus may be used to hold the X-ray generation mechanism 401 and adjust the position and posture of the X-ray generation mechanism 401. The X-ray generation mechanism 401 may include an X-ray tube generator and an X-ray beam limiter, the X-ray tube generator is configured to generate X-rays, and the X-ray beam limiter is configured to confine the X-rays generated by the X-ray tube generator within a predetermined range.

Further, the wall stand assembly 501 or the examination table 601 is used according to the site of a subject to be imaged that needs to be imaged and the state of the subject to be imaged, wherein the subject to be imaged may stand in front of the wall stand assembly 501 or lie on the examination table 601. After the subject to be imaged stands or lies down in position, the X-rays generated by the X-ray tube generator and collimated by the X-ray beam limiter penetrate a predetermined site of the subject to be imaged. Then, for example, an X-ray detector provided at the wall stand assembly 501 and the examination table 601 detects X-rays penetrating the subject to be imaged, the X-ray detector generates an output signal based on the intensity of the rays impacting each discrete region of the detector, the output signal is processed to generate an image that can be displayed for viewing, and the image can be displayed in a display apparatus of the X-ray imaging system. Thus, by utilizing the difference in the penetration capability of X-rays to different substances, the X-rays penetrating the subject to be imaged are detected and processed to finally obtain an image showing the internal configuration of the subject to be imaged.

To image the subject to be imaged at the wall stand assembly 501 or the examination table 601 by using the X-ray imaging system, as described above, the telescopic cylinder 101, the main body frame 201 and the angle adjustment mechanism 301 of the suspension apparatus need to drive the X-ray generation mechanism 401 to move to a desired position and maintain a desired posture. The telescopic cylinder 101 of the suspension apparatus is mainly used to achieve movement of the X-ray generation mechanism 401 in a direction perpendicular to a top wall of a building (e.g., vertical direction), and the telescopic cylinder 101 also needs to support the weights of both the angle adjustment mechanism 301 and the X-ray generation mechanism 401. Therefore, the telescopic cylinder 101 is required to have sufficient structural stability and rigidity while being able to smoothly achieve the above movement. Existing telescopic cylinders 101 designed to achieve the above functions typically have complex structures and high costs.

### SUMMARY

Based on the above problems of the prior art, an object of the present application is to provide a telescopic cylinder which has sufficient structural stability and rigidity while allowing smooth extension and collapse, with good operational stability, low susceptibility to failure, easy maintenance, and low space requirements.

Another object of the present application is to provide an X-ray imaging system comprising the above-mentioned telescopic cylinder.

To achieve the foregoing objects, the implementations of the present application may adopt the following technical solutions.

The implementations of the present application provide a telescopic cylinder, comprising:
a plurality of sleeves, wherein the plurality of sleeves are arranged in a nested configuration and central axes of the plurality of sleeves are parallel to each other, and each inner sleeve of the plurality of sleeves is configured to be slidably connected to an outer sleeve adjacent thereto such that each of the inner sleeves can move to an extended position and a collapsed position relative to the corresponding outer sleeve; and
guide assemblies, wherein each of the guide assemblies comprises rollers and a rail that are configured as a set, the rollers are provided on left and right sidewalls and rear walls of at least some of the inner sleeves and the rails are correspondingly provided on left and right sidewalls and rear walls of at least some of the outer sleeves, the rollers and the rail in the same guide assembly are provided on an outer wall surface of the inner sleeve and an inner wall surface of the corresponding outer sleeve, respectively, such that the rollers can roll on guide surfaces defined by the rail during sliding of the inner sleeve relative to the corresponding outer sleeve, thereby guiding movement of the inner sleeve relative to the outer sleeve.

In an optional solution, the rollers are provided only on the left and right sidewalls and the rear wall of each of the inner sleeves.

In another optional solution, the sleeves are configured as an integrally formed structure.

In another optional solution, the central axis of the innermost sleeve is positioned at a location closer to a front side of the telescopic cylinder relative to the central axis of the outermost sleeve.

In another optional solution, the central axis of each of the inner sleeves is positioned at a location closer to a front side relative to the central axis of the outer sleeve adjacent to the inner sleeve.

In another optional solution, the rail comprises:
support arms fixed to the outer sleeve, each of the support arms protruding relative to the outer sleeve toward the corresponding inner sleeve and extending linearly along an axial direction of the outer sleeve; and
guide plates fixedly mounted to the support arms, each guide plate having a guide surface extending along the axial direction of the outer sleeve.

In another optional solution, each sidewall of the inner sleeve is provided with at least one pair of rollers, the rail on each sidewall of the corresponding outer sleeve defines two guide surfaces arranged facing away from each other, and the rollers in each pair are respectively located on the two guide surfaces arranged facing away from each other, and
the rear wall of the inner sleeve is provided with at least one roller, the rail on the rear wall of the corresponding outer sleeve defines two guide surfaces arranged facing each other, and the at least one roller is located between the two guide surfaces arranged facing each other.

In another optional solution, at least some of the rails define two guide surfaces arranged facing away from each other, the inner sleeve is provided with a first pair of rollers and a second pair of rollers constituted by the rollers, the first pair of rollers and the second pair of rollers are arranged spaced apart in an axial direction of the inner sleeve, and the two rollers in each pair of rollers are arranged on both sides of the rail and are in rolling contact with the corresponding guide surfaces respectively.

In another optional solution, the telescopic cylinder further comprises first adjustment assemblies corresponding to the guide assemblies, wherein the first adjustment assemblies each comprise a first adjusting member, a second adjusting member, and a first adjusting rod;
the first adjusting member is configured to be oscillatingly mounted to the inner sleeve, and the first pair of rollers is rotatably mounted to the first adjusting member and is capable of oscillating with the first adjusting member;
the second adjusting member is configured to be oscillatingly mounted to the inner sleeve, and the second pair of rollers is rotatably mounted to the second adjusting member and is capable of oscillating with the second adjusting member; and
the first adjusting rod is connected to the first adjusting member and the second adjusting member, and the first adjusting member and the second adjusting member can be caused to oscillate toward or away from each other by operating the first adjusting rod.

In another optional solution, the first adjusting member comprises a first substrate and a first protrusion, the first pair of rollers is rotatably mounted to the first substrate and the first substrate is oscillatingly mounted to the inner sleeve, and the first protrusion protrudes from the first substrate;
the second adjusting member comprises a second substrate and a second protrusion, the second pair of rollers is rotatably mounted to the second substrate and the second substrate is oscillatingly mounted to the inner sleeve, and the second protrusion protrudes from the second substrate; and
the first adjusting rod extends along the axial direction of the inner sleeve, and is inserted through the first protrusion and threadingly engages with the second protrusion.

In another optional solution, at least some of the rails define two guide surfaces arranged facing each other, the inner sleeve is provided with a third pair of rollers constituted by the rollers, the two rollers in the third pair of rollers are both disposed between the two guide surfaces, and each roller is in rolling contact at least with one of the guide surfaces.

In another optional solution, the telescopic cylinder further comprises second adjustment assemblies corresponding to the guide assemblies, wherein the second adjustment assemblies each comprise a third adjusting member and a second adjusting rod;
the third adjusting member is configured to be oscillatingly mounted to the inner sleeve, and the third pair of rollers is rotatably mounted to the third adjusting member and is capable of oscillating with the third adjusting member; and
the second adjusting rod is connected to the third adjusting member, and the third adjusting member can be caused to oscillate by operating the second adjusting rod.

In another optional solution, the third adjusting member comprises a third substrate to which the third pair of rollers is rotatably mounted and which is oscillatingly mounted to the inner sleeve, and a third protrusion protruding from the third substrate; and
the second adjustment assembly further comprises a sleeve protrusion provided on the inner sleeve, the second adjusting rod extends in a direction perpendicular to the axial direction of the inner sleeve, and the second adjusting rod is inserted through the sleeve protrusion and threadingly engages with the third protrusion.

The present application further provides an X-ray imaging system as follows, which includes the telescopic cylinder according to any one of the above technical solutions.

In an optional solution, the X-ray imaging system further comprises a main body frame, an angle adjustment mechanism, and an X-ray generation mechanism,
wherein the outermost sleeve of the telescopic cylinder is mounted to the main body frame in a manner such that the outermost sleeve is rotatable about the central axis thereof, and
the innermost sleeve of the telescopic cylinder is connected to the X-ray generation mechanism via the angle adjustment mechanism, and the X-ray generation mechanism is configured to be capable of being positioned on a front side of the telescopic cylinder.

### BRIEF DESCRIPTION OF THE DRAWINGS

FIG. 1 is a schematic side view showing a partial structure of an X-ray imaging system according to an embodiment of the present application.
FIG. 2 and FIG. 3 are schematic side views showing an assembled unit of a telescopic cylinder and an angle adjustment mechanism applicable to the X-ray imaging system of FIG. 1, wherein the telescopic cylinder is in a collapsed state in FIG. 2, and the telescopic cylinder is in an extended state in FIG. 3.
FIG. 4 is a schematic enlarged partial view showing the telescopic cylinder in FIG. 2.
FIG. 5 is a schematic top view showing the telescopic cylinder in FIG. 2.
FIG. 6 is a schematic perspective view showing the outermost sleeve (integrally formed with support arms of rails) of the telescopic cylinder in FIG. 2.
FIG. 7 to FIG. 11 are schematic top views showing respective sleeves (integrally formed with the support arms of the rails) of the telescopic cylinder in FIG. 2.
FIG. 12 is a schematic enlarged view showing the structure of a rail on a sidewall of the outermost sleeve in FIG. 6.
FIG. 13 is a schematic enlarged view showing the structure of a rail on a rear wall of the outermost sleeve in FIG. 6.
FIG. 14 is a schematic perspective view showing an assembled unit of the innermost sleeve, rollers and adjustment assemblies of the telescopic cylinder in FIG. 2.
FIG. 15 is a schematic side view showing the assembled unit in FIG. 14, wherein a first pair of rollers and a second pair of rollers are in a state before being subjected to adjustment.
FIG. 16 is a schematic side view showing the assembled unit in FIG. 14, wherein the first pair of rollers and the second pair of rollers are in a state after being subjected to adjustment.
FIG. 17 is a schematic rear view showing the assembled unit in FIG. 14, wherein a third pair of rollers is in a state before being subjected to adjustment.
FIG. 18 is a schematic rear view showing the assembled unit in FIG. 14, wherein the third pair of rollers is in a state after being subjected to adjustment.
FIG. 19 is schematic top view showing a telescopic cylinder according to a second embodiment of the present application.
FIG. 20 is a schematic top view showing a telescopic cylinder according to a third embodiment of the present application.
FIG. 21 is a schematic top view showing a telescopic cylinder according to a fourth embodiment of the present application.
FIG. 22 is a schematic perspective view showing an existing X-ray imaging system.

### DETAILED DESCRIPTION

Embodiments of the present application are described below with reference to the accompanying drawings. For ease of understanding, elements shown in the drawings may include elements whose dimensions, scales and the like differ from actual dimensions, scales, and the like. Additionally, in order to provide a concise description in the specific description process of the embodiments, not all features of the embodiments are described in detail. For those of ordinary skill in the art related to the disclosure of the present application, some supplements and refinements, as well as design, manufacture, or production changes made on the basis of the technical content disclosed in the present application are common technical means and still fall within the scope of the present application, and should not be construed as the disclosure of the present application being insufficient.

Unless defined otherwise, the technical terms or scientific terms used in the claims and the description should have the usual meanings that are understood by those skilled in the art to which the present application belongs. Terms such as "first", "second", and similar terms used in the description and claims of the present application do not denote any order, quantity, or importance, but are only intended to distinguish different constituents. The word "include," "comprise," or a similar word is intended to mean that a component or an object that appears before "include" or "comprise" encompasses a component or an object and equivalent components that are listed after "include" or "comprise," and does not exclude other components or objects. The terms "connect" or "link" and similar words are not limited to physical or mechanical connections, and are not limited to direct or indirect connections.

In the present application, the use of the expression "substantially" intends to mean that a condition defined by such expression is satisfied within a reasonable error range recognized by those skilled in the art, and the expression has a similar meaning when it is used in the following description.

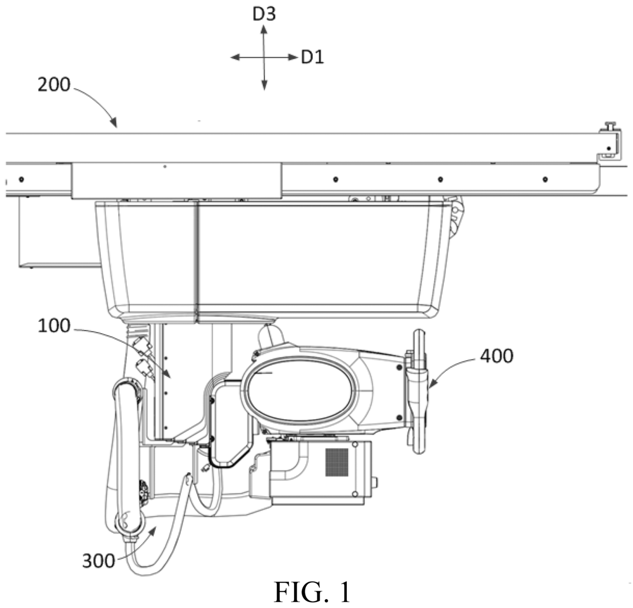

In the present application, unless otherwise specified, when an X-ray imaging system according to the present application is in an initial state (see FIG. 1), an X-ray generation mechanism is located on a front side of a telescopic cylinder and correspondingly the telescopic cylinder is located on a rear side of the X-ray generation mechanism. Thus, in the telescopic cylinder, a wall of each sleeve facing the front side, i.e. facing the X-ray generation mechanism, is a front wall and a wall of each sleeve opposite its front wall is a rear wall. Further, when the X-ray imaging system according to the present application is in the initial state, a left side and a right side refer to a left side and a right side when viewed in a direction toward the front side. Thus, in the telescopic cylinder, a wall of each sleeve facing the left side is a left sidewall and a wall of each sleeve facing the right side is a right sidewall. Further, an upper side and a lower side refer to an upper side and a lower side in a vertical direction. Additionally, "axial direction" refers to a direction along a central axis of the sleeve of the telescopic cylinder.

In the present application, unless otherwise specified, based on the "front side", "rear side", "left side", "right side", "upper side", and "lower side" explicitly defined above, a "front-rear direction", a "left-right direction", and an "up-down direction" respectively refer to front-rear, left-right, and up-down directions of the X-ray imaging system according to the present application, where the front-rear direction is a first direction described in the embodiments of the present application, the left-right direction is a second direction described in the embodiments of the present application, the up-down direction (vertical direction) is a third direction described in the embodiments of the present application, and the first direction, the second direction, and the third direction are mutually perpendicular.

The structure of an X-ray imaging system according to the embodiments of the present application, especially the structure of a telescopic cylinder of the X-ray imaging system, is described below with reference to the accompanying drawings.

As shown in FIG. 1, the X-ray imaging system according to an embodiment of the present application may include a suspension apparatus which includes a telescopic cylinder 100, a main body frame 200, an angle adjustment mechanism 300 and an X-ray generation mechanism 400.

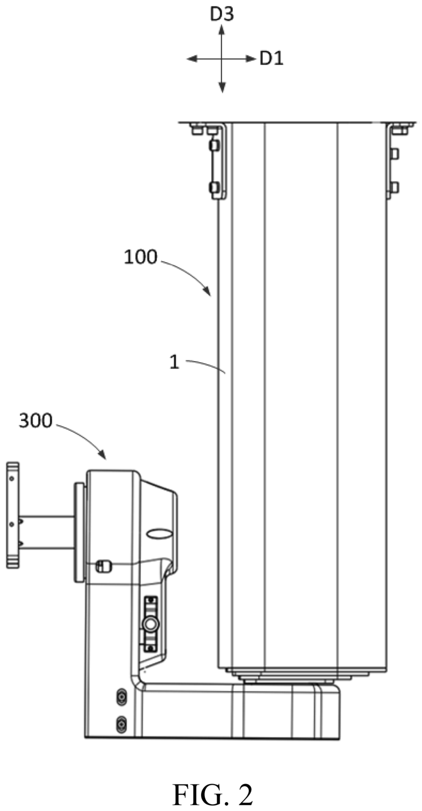

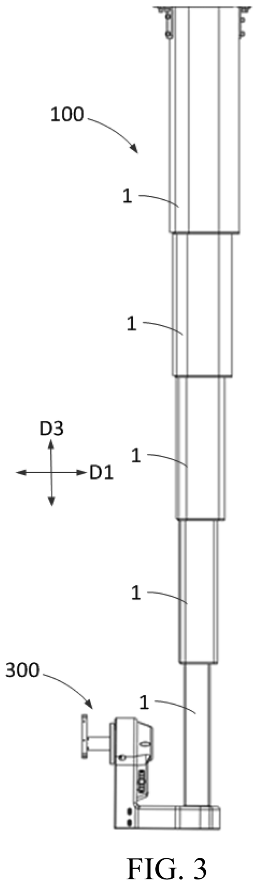

The main body frame 200 holds and supports the telescopic cylinder 100 and the angle adjustment mechanism 300, the main body frame 200 may include a rail mechanism constituted by a plurality of rails, and the plurality of rails of the rail mechanism can guide the main body frame 200 to translate in a predetermined plane, whereby the main body frame 200 can be positioned at any position within a predetermined range in the predetermined plane. The telescopic cylinder 100 may have a plurality of sleeves 1 (see FIG. 2 and FIG. 3), and the sleeves 1 are nested or sleeved together to form a controllable telescopic configuration. One end portion of the telescopic cylinder 100 is mounted to the main body frame 200 and the other end portion is connected to the angle adjustment mechanism 300 (see FIG. 2 and FIG. 3), and the telescopic cylinder 100 is rotatable relative to the main body frame 200. In this manner, the telescopic cylinder 100 is not only switchable between a collapsed state (see FIG. 2) and an extended state (see FIG. 3), thereby driving the angle adjustment mechanism 300 to reciprocate in a third direction D3, which is, for example, a vertical direction, but also capable of driving the angle adjustment mechanism 300 to rotate. The specific structure of the telescopic cylinder 100 will be described in more detail below. Furthermore, the angle adjustment mechanism 300 is further connected to the X-ray generation mechanism 400, and is primarily configured to adjust rotation angles of an X-ray generator and an X-ray beam limiter of the X-ray generation mechanism 400 rotating about an axis extending in a horizontal direction. The X-ray generation mechanism 400 may include the X-ray generator and the X-ray beam limiter. The X-ray generator may include a tube assembly capable of generating X-rays. The X-ray beam limiter confines the X-rays generated by the X-ray generator within a predetermined range, thereby enabling the X-rays to be concentrated at a predetermined site of a subject to be imaged. The present application does not limit the specific configuration of the X-ray generator, and the X-ray generator may use various existing or future technologies. The X-ray generator and the X-ray beam limiter may be mounted to, for example, a ceiling (an indoor top surface) of a building or the like via the suspension apparatus, and the suspension apparatus can be used to hold the X-ray generator and the X-ray beam limiter and adjust the positions and postures of the X-ray generator and the X-ray beam limiter. Thus, the telescopic cylinder 100, the main body frame 200 and the angle adjustment mechanism 300 of the suspension apparatus can move in a predetermined range in multiple spatial degrees of freedom, thereby driving the X-ray generator and the X-ray beam limiter to move correspondingly.

Thus, after the subject to be imaged, such as a patient, for example, stands or lies down in position, the subject to be imaged may remain stationary, and under the control of a control unit, the X-ray generator and the X-ray beam limiter of the X-ray generation mechanism 400 can move to a desired position, so that the X-ray imaging system can be used to perform desired three-dimensional imaging on the subject to be imaged.

The specific configuration of a telescopic cylinder 100 according to a first embodiment of the present application, which is applicable to the above-described X-ray imaging system, will be specifically described below with reference to the accompanying drawings.

As shown in FIG. 2 to FIG. 5, the telescopic cylinder 100 according to the embodiment of the present application includes a plurality of (five in the present embodiment) sleeves 1, guide assemblies 2, a first adjustment assembly 3 and a second adjustment assembly 4 assembled together.

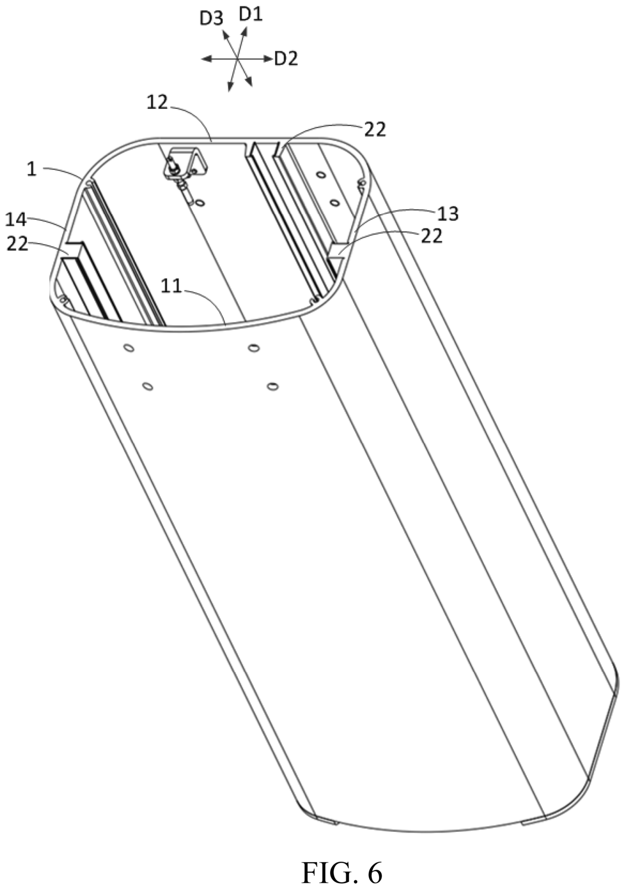

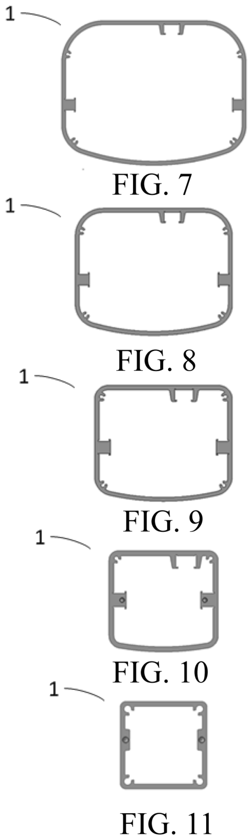

In the present embodiment, the sleeves 1 may be made of a metallic material such as aluminum or an aluminum alloy, and the sleeves 1 are configured to have an integrally formed structure, for example, formed by extrusion molding. To enable the plurality of sleeves 1 to be assembled together in a space-saving manner, the plurality of sleeves 1 have similar configurations in terms of structure and shape. Specifically, in one aspect, as shown in FIG. 7 to FIG. 11, the plurality of sleeves 1 have substantially the same cross-sectional shape although the cross-sectional dimensions of the plurality of sleeves 1 are different. Specifically, in the present embodiment, each sleeve 1 has a substantially rectangular cross-sectional shape. In another aspect, the plurality of sleeves 1 have substantially the same axial length. Further, as shown in FIG. 6, taking the outermost sleeve 1 of the telescopic cylinder 100 after completion of the assembly as an example, each sleeve 1 has a front wall 11, a rear wall 12, a left sidewall 13 and a right sidewall 14 connected to one another. The front wall 11 is connected to the front ends of the left sidewall 13 and the right sidewall 14, respectively, and the rear wall 12 is connected to the rear ends of the left sidewall 13 and the right sidewall 14, respectively, whereby they enclose and define a cavity with both axial ends open toward the outside, such that the sleeve 1 is formed into a hollow cylindrical structure.

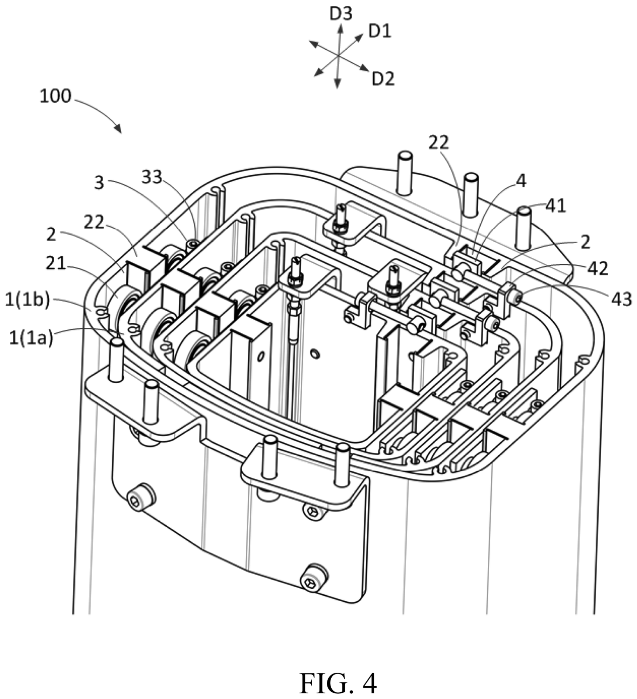

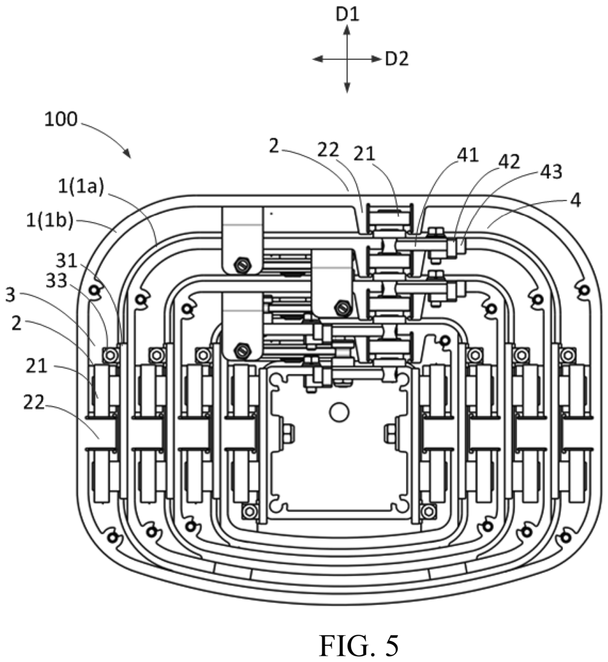

In the case where all the sleeves 1 are each formed into a hollow cylindrical structure, as shown in FIG. 4 and FIG. 5, the plurality of sleeves 1 are arranged in a nested configuration. That is, the sleeves 1 located on the inner side (referred to as inner sleeves 1a in the present application) are all received within the cavities of the sleeves 1 located outside thereof and adjacent thereto (referred to as outer sleeves 1b in the present application). It can be understood that in the present application, the terms inner sleeve 1a and outer sleeve 1b are relative, and except that after the telescopic cylinder 100 is assembled, the sleeve 1 located on the innermost side (referred to as the innermost sleeve in the present application) can only function as the inner sleeve 1a and the sleeve 1 located on the outermost side (referred to as the outermost sleeve in the present application) can only function as the outer sleeve 1b, the remaining sleeves 1 can function as either inner sleeves 1a or outer sleeves 1b. Whether the sleeves 1 function as inner sleeves 1a or outer sleeves 1b depends on the specific situation. Moreover, in the present application, an inner sleeve 1a and an outer sleeve 1b adjacent to the inner sleeve 1a are paired with each other, and once there is a sleeve 1 that functions as an inner sleeve 1a, there must be an outer sleeve 1b adjacent to the inner sleeve 1a; and vice versa. In addition, referring to FIG. 1, the outermost sleeve of the telescopic cylinder 100 may be mounted to the main body frame 200 via a bearing so as to be rotatable about the central axis thereof. In this manner, the entire telescopic cylinder 100 is rotatable about the central axis of the outermost sleeve relative to the main body frame 200. The innermost sleeve of the telescopic cylinder 100 is connected to the X-ray generation mechanism 400 via the angle adjustment mechanism 300. In this manner, the telescopic movement of the telescopic cylinder 100 can adjust the position of the X-ray generation mechanism 400 in the third direction D3 via the angle adjustment mechanism 300. In addition, the innermost sleeve of the telescopic cylinder 100 may be non-rotatably connected to the angle adjustment mechanism, such that the X-ray generation mechanism 400 may always be positioned on the front side of the telescopic cylinder 100. In an optional solution, the innermost sleeve of the telescopic cylinder 100 may be rotatably connected to the angle adjustment mechanism, which enables more flexible adjustment of the position of the X-ray generation mechanism 400.

After all the sleeves 1 are assembled by nesting, the central axes of all the sleeves 1 are parallel to each other. As shown in FIG. 5, the central axis of each inner sleeve 1a is positioned at a location closer to a front side (i.e., the lower side in FIG. 5) relative to the central axis of the outer sleeve 1b adjacent to the inner sleeve 1a. In this manner, in each pair of the inner sleeve 1a and the outer sleeve 1b, the central axis of the inner sleeve 1a is positioned at a location closer to a front side relative to the central axis of the outer sleeve 1b, such that the central axis of the innermost sleeve is positioned at a location significantly closer to the front side relative to the central axis of the outermost sleeve. In this manner, the distance between the central axis of the innermost sleeve and a front end of the X-ray generation mechanism 400 can be shortened, which leads to a space-saving layout; and moreover, since the structure where the X-ray generation mechanism 400 is connected to the telescopic cylinder 100 via the angle adjustment mechanism 300 actually forms a cantilever beam configuration (see FIG. 1), a load moment generated by the angle adjustment mechanism 300 and the X-ray generation mechanism 400 becomes relatively small, which is beneficial to the structural stability of the cantilever beam configuration constituted by the telescopic cylinder 100, the angle adjustment mechanism 300 and the X-ray generation mechanism 400, and can improve the operational stability of the entire X-ray imaging system. Furthermore, in the present embodiment, as shown in FIG. 5, the central axes of all the sleeves 1 may be located in the same plane, which can improve the left-right balance of the overall structure of the telescopic cylinder 100.

To enable the telescopic cylinder 100 to be extended and collapsed along the third direction D3, each inner sleeve 1a of the plurality of sleeves 1 is configured to be slidably connected to an outer sleeve 1b adjacent thereto, such that each inner sleeve 1a is movable to an extended position and a collapsed position relative to the corresponding outer sleeve 1b. When all the inner sleeves 1a have moved to the extended positions relative to the corresponding outer sleeves 1b, the entire telescopic cylinder 100 is in an extended state, and the length of the entire telescopic cylinder 100 is slightly less than or substantially equal to the sum of the axial lengths of the sleeves 1. When all the inner sleeves 1a have moved to the collapsed positions relative to the corresponding outer sleeves 1b, as shown in FIG. 1 and FIG. 2, all the sleeves 1 are arranged to overlap in the third direction D3, the entire telescopic cylinder 100 is in a collapsed state, and the length of the entire telescopic cylinder 100 is substantially equal to the axial length of the outermost sleeve. It can be understood that the telescopic cylinder 100 of the present application can move and maintain in an intermediate state between the extended state and the collapsed state, without having to be in the above-mentioned extended and collapsed states. To enable extension and collapse of the telescopic cylinder 100, a driving motor and a transmission member such as a transmission chain or transmission cable driven by the driving motor may be provided on the main body frame 200, and the transmission member is connected to all the sleeves 1 respectively. In the present embodiment, except for the innermost sleeve and the outermost sleeve, a cable sheave for engaging with the transmission member is provided at a lower portion of an inner wall surface of each of the remaining sleeves 1, and the cable sheave is freely rotatable relative to the sleeve 1. The transmission member may be wound from one side of the cable sheave, through the bottom of the cable sheave, to the other side of the cable sheave. In this manner, during a process of driving the transmission member, the cable sheaves can function as movable pulleys, thereby driving the sleeves 1 connected thereto to move up and down, and accordingly enabling extension and collapse of the entire telescopic cylinder 100. Furthermore, to prevent the transmission member from unexpectedly disengaging from the cable sheave during transmission, a plurality of (two in the present embodiment) limiting portions may be provided at positions on the sleeve 1 close to an outer peripheral edge of the cable sheave, and these limiting portions can sufficiently prevent the transmission member from disengaging from the cable sheave. It can be understood that, to achieve synchronized movement of all the sleeves 1 except the outermost sleeve, various solutions in the prior art in which the transmission member is connected to the sleeves 1 may be employed, and no further detailed description is provided here.

In the present embodiment, to enable smooth and stable movement of the sleeves 1 during telescoping of the telescopic cylinder 100, as shown in FIG. 4 and FIG. 5, guide assemblies 2 are provided between the inner sleeve 1a and the outer sleeve 1b adjacent to each other. Specifically, in the present embodiment, the guide assemblies 2 are provided between the left and right sidewalls of the inner sleeve 1a and the left and right sidewalls of the outer sleeve 1b, and between the rear wall 12 of the inner sleeve 1a and the rear wall 12 of the outer sleeve 1b. Each guide assembly 2 includes rollers 21 and a rail 22 that are configured as a set, the rollers 21 are provided on the left and right sidewalls and the rear wall 12 of each inner sleeve 1a, and the rails 22 are correspondingly provided on the left and right sidewalls and the rear wall 12 of each outer sleeve 1b. Specifically, the rollers 21 and the rail 22 in the same guide assembly 2 are respectively disposed on an outer wall surface of the inner sleeve 1a and an inner wall surface of the corresponding outer sleeve 1b, such that the rollers 21 can roll on guide surfaces defined by the rail 22 during sliding of the inner sleeve 1a relative to the corresponding outer sleeve 1b, thereby guiding the movement of the inner sleeve 1a relative to the outer sleeve 1b, preventing the inner sleeve 1a and the outer sleeve 1b from being skewed during the relative movement, and thus avoiding undesirable failure of the telescopic cylinder 100.

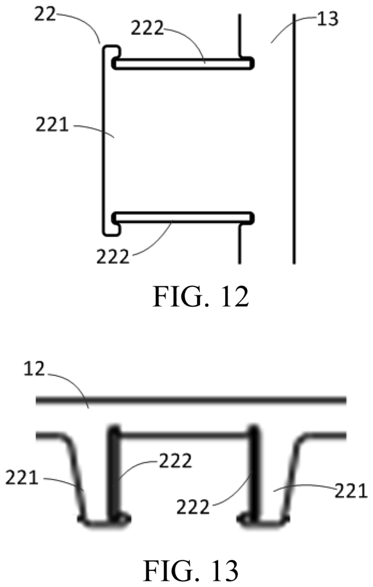

As shown in FIG. 12 and FIG. 13, the rail 22 includes support arms 221 fixed to the sleeve 1 (e.g., integrally formed) and guide plates 222 provided on the support arms 221. The support arms 221 are fixed to the outer sleeve 1b, and each support arm 221 protrudes toward the corresponding inner sleeve 1a relative to the outer sleeve 1b and extends linearly along the axial direction of the outer sleeve 1b. It can be understood that the number of support arms 221 of each rail 22 and the shape and size of the support arm 221 can be adjusted as needed, which will be illustrated with examples below. In the case where the support arm 221 is formed integrally with the sleeve 1 and made of a metallic material such as aluminum or an aluminum alloy, the material properties of the support arm 221 are not particularly suitable for guiding rolling of the roller 21. Therefore, the guide plates 222 made of, for example, steel are provided. The guide plates 222 may be fixedly mounted to the support arms 221 by means of snap-fitting and/or bonding, and the guide plate 222 has a guide surface extending along the axial direction of the outer sleeve 1b.

Further, in consideration of the differences in the space sizes between different portions of the inner sleeve 1a and different portions of the outer sleeve 1b and the differences in the dimensions of these portions, the guide assemblies 2 provided between the left and right sidewalls of the inner sleeve 1a and the left and right sidewalls of the outer sleeve 1b are structurally different from the guide assembly 2 provided between the rear wall 12 of the inner sleeve 1a and the rear wall 12 of the outer sleeve 1b.

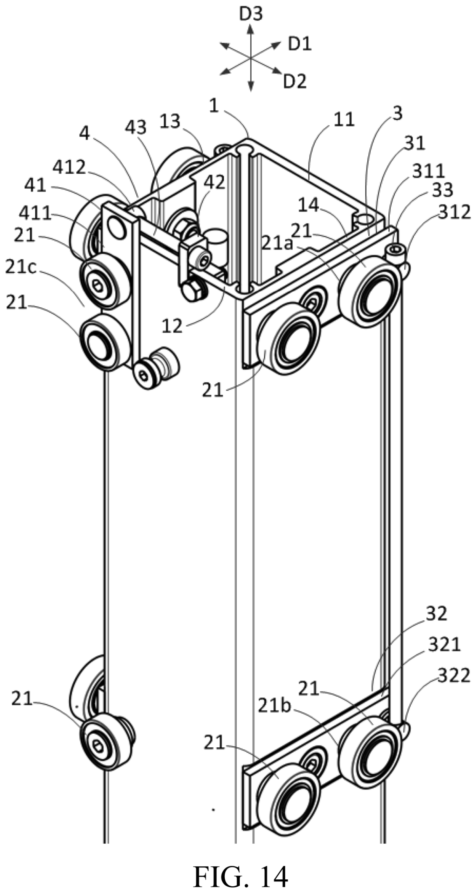

First, as shown in FIG. 4 to FIG. 13, regarding the guide assembly 2 between the left sidewall 13 of the inner sleeve 1a and the left sidewall 13 of the outer sleeve 1b and the guide assembly 2 between the right sidewall 14 of the inner sleeve 1a and the right sidewall 14 of the outer sleeve 1b, each of the rails 22 has one support arm 221 with a larger thickness in a first direction D1, each of the front and rear side surfaces of the support arm 221 is provided with the guide plate 222, and the two guide plates 222 respectively define guide surfaces. In this manner, the rail 22 actually defines two guide surfaces arranged facing away from each other. As shown in FIG. 14 to FIG. 16, the inner sleeve 1a is provided with a first pair of rollers 21a and a second pair of rollers 21b constituted by the rollers 21 corresponding to the rail 22, and each pair of rollers includes two rollers 21 arranged spaced apart in the first direction D1. Furthermore, the first pair of rollers 21a and the second pair of rollers 21b are arranged spaced apart in the axial direction of the inner sleeve 1a, and the two rollers 21 in each pair of rollers are disposed on both sides of the rail 22 and are in rolling contact with the corresponding guide surfaces, respectively. In this manner, by providing the guide assembly 2 including one rail 22 and the rollers 21 on both sides of the rail 22, the rigidity of the entire telescopic cylinder 100 in the first direction D1 can be improved, while ensuring that the telescopic cylinder 100 is extended and collapsed smoothly and stably. That is, in the case where the telescopic cylinder 100, the angle adjustment mechanism 300, and the X-ray generation mechanism 400 constitute a cantilever beam structure, the telescopic cylinder 100 can support the angle adjustment mechanism 300 and the X-ray generation mechanism 400 stably while ensuring that the telescopic cylinder 100 is extended and collapsed smoothly and stably.

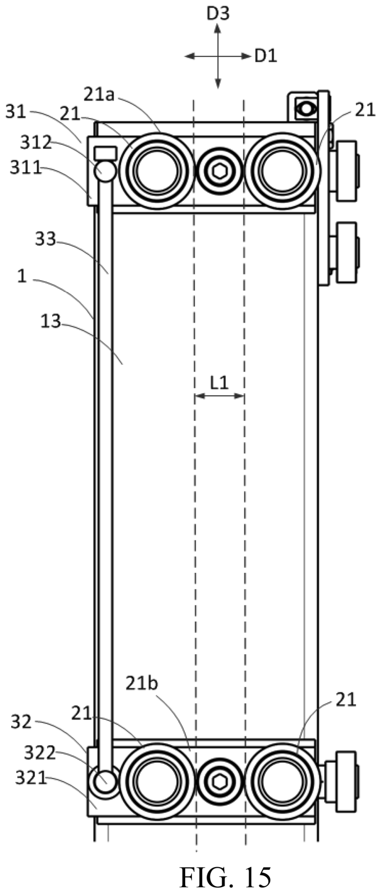

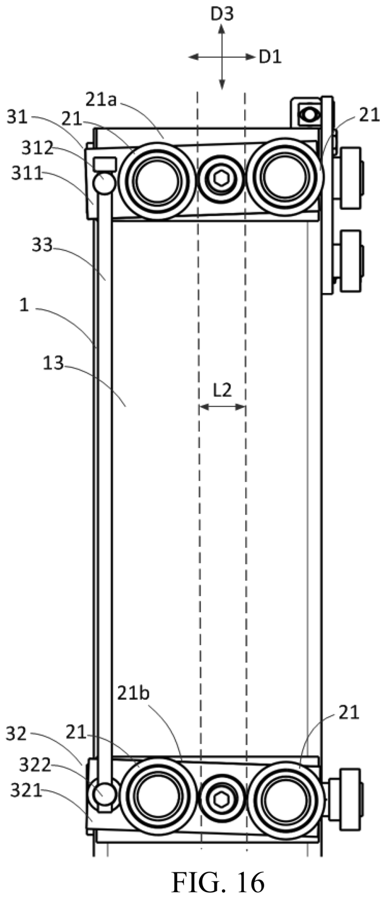

Furthermore, to ensure that the paired rollers 21 in the guide assembly 2 can really be in rolling contact with the guide surfaces of the rail 22, a first adjustment assembly 3 is provided corresponding to each of the guide assemblies 2. As shown in FIG. 14 to FIG. 16, the first adjustment assembly 3 includes a first adjusting member 31, a second adjusting member 32 and a first adjusting rod 33. Specifically, the first adjusting member 31 includes a first substrate 311 and a first protrusion 312, the first pair of rollers 21a is rotatably mounted to the first substrate 311, and the first substrate 311 is oscillatingly mounted to the left sidewall 13 or the right sidewall 14 of the inner sleeve 1a. The first substrate 311 is capable of oscillating about a portion thereof connected to the inner sleeve 1a within a predetermined range relative to the inner sleeve 1a, the two rollers 21 in the first pair of rollers 21a are located on a front side and a rear side of an oscillation center of the first substrate 311, and the two rollers 21 are freely rotatable relative to the first substrate 311. In addition, the first protrusion 312 is disposed at one end of the first substrate 311 and protrudes from the first substrate 311 in a direction away from the first substrate 311. Thus, the first adjusting member 31 is configured to be oscillatingly mounted to the inner sleeve 1a, and the first pair of rollers 21a is rotatably mounted to the first adjusting member 31 and is capable of oscillating with the first adjusting member 31. The second adjusting member 32 includes a second substrate 321 and a second protrusion 322, the second pair of rollers 21b is rotatably mounted to the second substrate 321, and the second substrate 321 is oscillatingly mounted to the left sidewall 13 or the right sidewall 14 of the inner sleeve 1a. The second substrate 321 is capable of oscillating about a portion thereof connected to the inner sleeve 1a within a predetermined range relative to the inner sleeve 1a, the two rollers 21 in the second pair of rollers 21b are located on a front side and a rear side of an oscillation center of the second substrate 321, and the two rollers 21 are freely rotatable relative to the second substrate 321. In addition, the second protrusion 322 is disposed at one end of the second substrate 321, and the second protrusion 322 protrudes from the second substrate 321 in a direction away from the second substrate 321. Thus, the second adjusting member 32 is configured to be oscillatingly mounted to the inner sleeve 1a, and the second pair of rollers 21b is rotatably mounted to the second adjusting member 32 and is capable of oscillating with the second adjusting member 32. Further, the first adjusting rod 33 extends along the axial direction of the inner sleeve 1a, and is inserted through the first protrusion 312 and threadingly engages with the second protrusion 322. The first adjusting member 31 and the second adjusting member 32 can be caused to oscillate toward or away from each other by operating the first adjusting rod 33 (when viewed in the second direction, the first adjusting member 31 and the second adjusting member 32 rotate in opposite directions). That is, the first substrate 311 of the first adjusting member 31 and the second substrate 321 of the second adjusting member 32 are caused to oscillate such that their same side ends move close to or away from each other. In this process, the distance in the first direction D1 between the two rollers 21 in the first pair of rollers 21a and the distance in the first direction D1 between the two rollers 21 in the second pair of rollers 21b can be adjusted simultaneously (the distance L2 in FIG. 16 is less than the distance L1 in FIG. 15), so that the two rollers 21 of the first pair of rollers 21a and the two rollers 21 of the second pair of rollers 21b are really in contact with the two guide surfaces of the rail 22.

Secondly, as shown in FIG. 4 to FIG. 13, regarding the guide assembly 2 between the rear wall 12 of the inner sleeve 1a and the rear wall 12 of the outer sleeve 1b, the rail 22 has two support arms 221 spaced apart in the second direction D2, the guide plate 222 are each provided on the side surfaces of the two support arms 221 facing each other, and the two guide plates 222 respectively define guide surfaces. In this manner, the rail 22 actually defines two guide surfaces arranged facing each other. In correspondence with the rail 22, as shown in FIGS. 14, 17 and 18, the inner sleeve 1a is provided with a third pair of rollers 21c constituted by the rollers 21, the two rollers 21 of the third pair of rollers 21c are each provided between the two support arms 221, and each roller 21 is in rolling contact with at least one of the two guide surfaces. In addition, in addition to the third pair of rollers 21c, another roller 21 spaced apart from the third pair of rollers 21c in the third direction D3 may further be provided, which can improve the guiding function of the guide assembly 2.

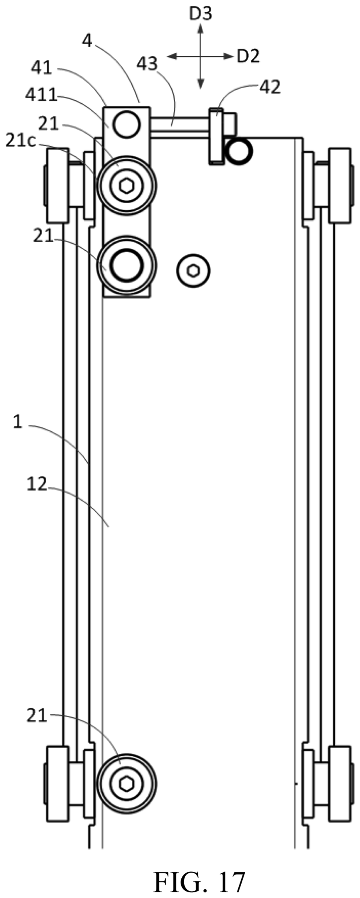

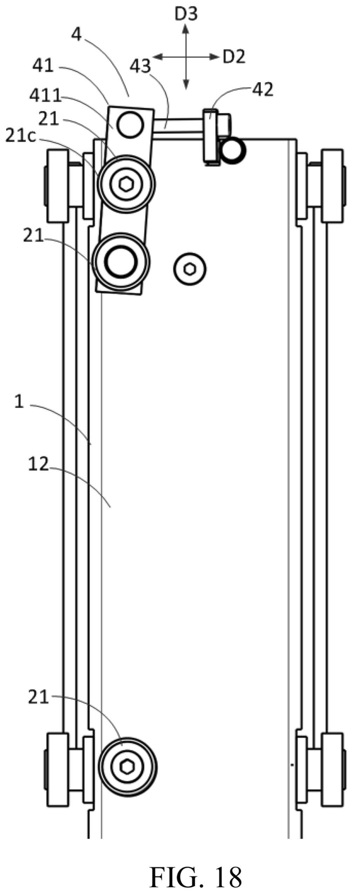

Furthermore, to ensure that the rollers 21 of the third pair of guide wheels in the guide assembly 2 can really be in rolling contact with at least one guide surface of the rail 22, a second adjustment assembly 4 is provided corresponding to each of the guide assemblies 2. The second adjustment assembly 4 includes a third adjusting member 41, a sleeve protrusion 42 and a second adjusting rod 43. Specifically, as shown in FIG. 14, FIG. 17 and FIG. 18, the third adjusting member 41 includes a third substrate 411 and a third protrusion 412, the third pair of rollers 21c is rotatably mounted to the third substrate 411 and the third substrate 411 is oscillatingly mounted to the inner sleeve 1a. The third substrate 411 is capable of oscillating about a portion thereof connected to the inner sleeve 1a within a predetermined range relative to the inner sleeve 1a, the two rollers 21 of the third pair of rollers 21c are located on both upper and lower sides of an oscillation center of the third substrate 411, and the two rollers 21 are freely rotatable relative to the third substrate 411. The third adjusting member 41 is configured to be oscillatingly mounted to the inner sleeve 1a, and the third pair of rollers 21c is rotatably mounted to the adjusting member and is capable of oscillating with the third adjusting member 41. Further, the sleeve protrusion 42 is provided on the inner sleeve 1a, and the second adjusting rod 43 extends in a direction perpendicular to the axial direction of the inner sleeve 1a, and is inserted through the sleeve protrusion 42 and threadingly engages with the third protrusion 412. The third adjusting member 41 can be caused to oscillate by operating the second adjusting rod 43, so that the two rollers 21 of the third pair of rollers 21c are slightly offset in the second direction D2 (see the change in the relative positions of the two rollers 21 in the second direction D2 in FIG. 18 relative to the relative positions of the two rollers 21 in the second direction D2 in FIG. 17), thereby causing the two rollers 21 of the third pair of rollers 21c to really be in rolling contact with the two guide surfaces of the rail 22, respectively.

It can be understood that, on the one hand, since there is no large load on both sides of the telescopic cylinder 100 in the second direction D2, and on the other hand, to facilitate positioning the central axis of the innermost sleeve to be closer to the front side than the central axis of the outermost sleeve, the guide assembly 2 may be provided only between the left and right sidewalls of the inner sleeve 1a and the left and right sidewalls of the outer sleeve 1b, and between the rear wall 12 of the inner sleeve 1a and the rear wall 12 of the outer sleeve 1b. That is, no guide assembly 2 is provided between the front wall 11 of the inner sleeve 1a and the front wall 11 of the outer sleeve 1b. It can be understood that, by means of the positioning function of the guide assembly 2 provided between the left and right sidewalls of the inner sleeve 1a and the left and right sidewalls of the outer sleeve 1b, a small gap may be provided between the front wall 11 of the inner sleeve 1a and the front wall 11 of the outer sleeve 1b, thereby avoiding undesired friction between the inner sleeve 1a and the outer sleeve 1b during telescoping of the telescopic cylinder 100.

By adopting the above solution, a telescopic cylinder 100 is provided, which has sufficient structural stability and rigidity while allowing smooth extension and collapse, with good operational stability and low susceptibility to failure, and thus has high reliability and practicability. Moreover, the telescopic cylinder 100 of the above embodiment is relatively uncomplicated in structure, with a compact structure and low space requirements, thus leading to low costs and ease of maintenance.

The specific structures of telescopic cylinders 100 according to other embodiments of the present application will be described below with reference to the accompanying drawings.

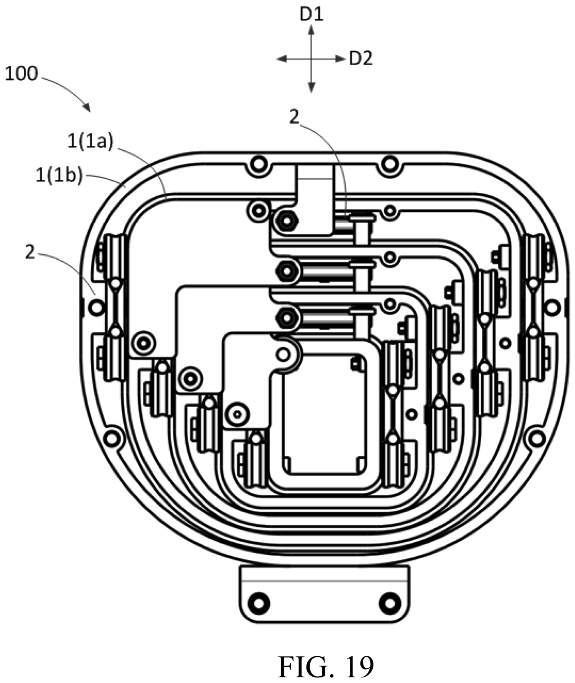

As shown in FIG. 19, a telescopic cylinder 100 according to a second embodiment of the present application is basically structurally the same as the telescopic cylinder 100 according to the first embodiment of the present application, and the differences between the two are mainly described below. In the present embodiment, after assembly of the telescopic cylinder 100 is completed, a front wall 11 of a sleeve 1 closer to the outer side has a more pronounced curved appearance. Moreover, different guide assemblies 2 arranged between left and right sidewalls of an inner sleeve 1a and left and right sidewalls of an outer sleeve 1b are arranged in a staggered manner in a first direction D1 to adapt to changes in the cross-sectional shape of the sleeve 1. Furthermore, no guide assembly 2 is provided between rear walls 12 of the outermost sleeve and the inner sleeve 1a connected thereto, and guide surfaces of all the guide assemblies 2 are defined by cylindrical guide rods. Correspondingly, the rollers 21 may have outer grooves matching the guide rods. In the present embodiment, the same effects as those described in the first embodiment can be achieved.

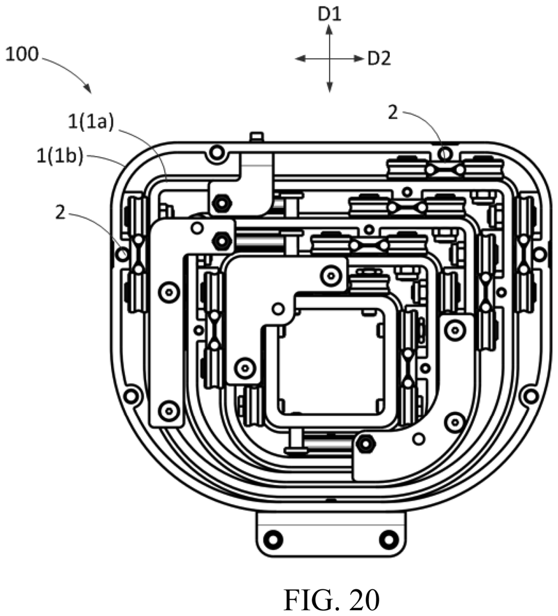

As shown in FIG. 20, a telescopic cylinder 100 according to a third embodiment of the present application is basically structurally the same as the telescopic cylinder 100 according to the second embodiment of the present application, and the differences between the two are mainly described below. Guide assemblies 2 are provided between rear walls 12 of all inner sleeves 1a and rear walls 12 of corresponding outer sleeves 1b and between left and right sidewalls of all inner sleeves 1a and left and right sidewalls of corresponding outer sleeves 1b, and the configuration of the guide assemblies 2 provided between the rear walls 12 of the inner sleeves 1a and the rear walls 12 of the outer sleeves 1b is the same as the configuration of the guide assemblies 2 provided between the left and right sidewalls of the inner sleeves 1a and the left and right sidewalls of the outer sleeves 1b of the telescopic cylinder 100, thereby improving the rigidity of the telescopic cylinder 100 in a second direction D2 and thus enhancing the supporting capability. Moreover, the different guide assemblies 2 arranged between the rear walls 12 of the inner sleeves 1a and the rear walls 12 of the outer sleeves 1b are arranged in a staggered manner in the second direction D2. In the present embodiment, the same effects as those described in the first embodiment can be achieved.

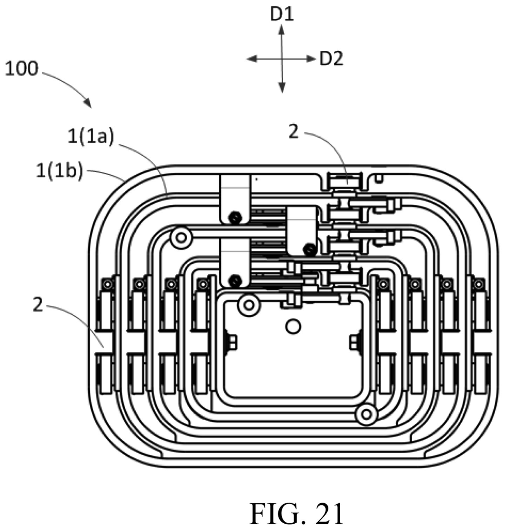

As shown in FIG. 21, a telescopic cylinder 100 according to a fourth embodiment of the present application is basically structurally the same as the telescopic cylinder 100 according to the first embodiment of the present application, and the differences between the two are mainly described below. In the present embodiment, the cross-sectional shape of each of sleeves 1 is formed in a rectangular shape having a smaller dimension in a first direction D1, and thus the space occupied by the telescopic cylinder 100 can be reduced in the first direction D1. In the present embodiment, the same effects as those described in the first embodiment can be achieved.

It should be understood that the above embodiments are only exemplary and are not intended to limit the present application. Those skilled in the art may make various modifications and changes to the above embodiments under the teachings of the present disclosure, without departing from the scope of the present application. The technical solutions of the present application are supplementarily described as follows.
i. It can be understood that in different embodiments of the present application and variants thereof, the guide assemblies 2 between some of the inner sleeves 1a and the corresponding outer sleeves 1b may be omitted as needed, as long as the object of the present application can be achieved.
ii. It can be understood that in different embodiments of the present application and variants thereof, the adjustment assemblies corresponding to the guide assemblies 2 may be omitted, as long as the rollers 21 of the guide assemblies 2 can really be in rolling contact with the guide surfaces of the rail 22.

Furthermore, in the above specific embodiments, the adjustment assembly is provided with an adjusting member that can oscillate relative to the sleeve 1. To prevent the adjusting member from oscillating undesirably relative to the sleeve 1, a locking member (e.g., a locking bolt) may be provided at the oscillation center position of the adjusting member, so that the relative positions of the adjusting plate and the sleeve 1 can be locked as needed to prevent the adjusting plate from oscillating undesirably relative to the sleeve 1.

iii. It can be understood that in the solution of the telescopic cylinder 100 according to the present application, in order to limit the range of relative movement between the adjacent sleeves 1, a limiting mechanism may be provided to achieve such a function.

iv. It can be understood that decorative members for decoration may be provided on the outer wall surfaces of all the sleeves 1 to improve the appearance of the entire telescopic cylinder 100.

It can be understood that some of the various components, structures, and constituent parts described above may be omitted without affecting the achievement of one or more objects of the present application. Different embodiments, examples or aspects may be appropriately combined, provided that they do not conflict or contradict each other.

The exemplary embodiments and variants of the present application have been described above; however, it should be understood that various modifications may be made. For example, same, similar, or other suitable results can be achieved if the described techniques are executed in a different order and/or if components in the described systems, architectures, devices, or circuits are combined in different ways and/or replaced or supplemented by additional components or equivalents thereof; and these changes or modifications also fall within the scope of protection of the claims.

## Claims

1. A telescopic cylinder, **characterized by** comprising:
a plurality of sleeves, wherein the plurality of sleeves are arranged in a nested configuration and central axes of the plurality of sleeves are parallel to each other, and each inner sleeve of the plurality of sleeves is configured to be slidably connected to an outer sleeve adjacent thereto such that each of the inner sleeves can move to an extended position and a collapsed position relative to the corresponding outer sleeve; and
guide assemblies, wherein each of the guide assemblies comprises rollers and a rail that are configured as a set, the rollers are provided on left and right sidewalls and rear walls of at least some of the inner sleeves and the rails are correspondingly provided on left and right sidewalls and rear walls of at least some of the outer sleeves, the rollers and the rail in the same guide assembly are provided on an outer wall surface of the inner sleeve and an inner wall surface of the corresponding outer sleeve, respectively, such that the rollers can roll on guide surfaces defined by the rail during sliding of the inner sleeve relative to the corresponding outer sleeve, thereby guiding movement of the inner sleeve relative to the outer sleeve.

2. The telescopic cylinder according to claim 1, wherein the rollers are provided only on the left and right sidewalls and the rear wall of each of the inner sleeves.

3. The telescopic cylinder according to claim 1, wherein the sleeves are configured as an integrally formed structure.

4. The telescopic cylinder according to any one of claims 1 to 3, wherein the central axis of the innermost sleeve is positioned at a location tube closer to a front side of the telescopic cylinder relative to the central axis of the outermost sleeve.

5. The telescopic cylinder according to claim 4, wherein the central axis of each of the inner sleeves is positioned at a location closer to a front side relative to the central axis of the outer sleeve adjacent to the inner sleeve.

6. The telescopic cylinder according to any one of claims 1 to 3, wherein the rail comprises:
support arms fixed to the outer sleeve, each of the support arms protruding relative to the outer sleeve toward the corresponding inner sleeve and extending linearly along an axial direction of the outer sleeve; and
guide plates fixedly mounted to the support arms, the guide plates each having a guide surface extending along the axial direction of the outer sleeve.

7. The telescopic cylinder according to any one of claims 1 to 3, wherein
each sidewall of the inner sleeve is provided with at least one pair of rollers, the rail on each sidewall of the corresponding outer sleeve defines two guide surfaces arranged facing away from each other, and the rollers in each pair are respectively located on the two guide surfaces arranged facing away from each other, and
the rear wall of the inner sleeve is provided with at least one roller, the rail on the rear wall of the corresponding outer sleeve defines two guide surfaces arranged facing each other, and the at least one roller is located between the two guide surfaces arranged facing each other.

8. The telescopic cylinder according to any one of claims 1 to 3, wherein at least some of the rails define two guide surfaces arranged facing away from each other, the inner sleeve is provided with a first pair of rollers and a second pair of rollers constituted by the rollers, the first pair of rollers and the second pair of rollers are arranged spaced apart in an axial direction of the inner sleeve, and the two rollers in each pair of rollers are arranged on both sides of the rail and are respectively in rolling contact with the corresponding guide surfaces.

9. The telescopic cylinder according to claim 8, further comprising first adjustment assemblies corresponding to the guide assemblies, wherein the first adjustment assemblies each comprise a first adjusting member, a second adjusting member, and a first adjusting rod;
the first adjusting member is configured to be oscillatingly mounted to the inner sleeve, and the first pair of rollers is rotatably mounted to the first adjusting member and is capable of oscillating with the first adjusting member;
the second adjusting member is configured to be oscillatingly mounted to the inner sleeve, and the second pair of rollers is rotatably mounted to the second adjusting member and is capable of oscillating with the second adjusting member; and
the first adjusting rod is connected to the first adjusting member and the second adjusting member, and the first adjusting member and the second adjusting member can be caused to oscillate toward or away from each other by operating the first adjusting rod.

10. The telescopic cylinder according to claim 9, wherein
the first adjusting member comprises a first substrate and a first protrusion, the first pair of rollers is rotatably mounted to the first substrate and the first substrate is oscillatingly mounted to the inner sleeve, and the first protrusion protrudes from the first substrate;
the second adjusting member comprises a second substrate and a second protrusion, the second pair of rollers is rotatably mounted to the second substrate and the second substrate is oscillatingly mounted to the inner sleeve, and the second protrusion protrudes from the second substrate; and
the first adjusting rod extends along the axial direction of the inner sleeve, and is inserted through the first protrusion and threadingly engages with the second protrusion.

11. The telescopic cylinder according to any one of claims 1 to 3, wherein at least some of the rails define two guide surfaces arranged facing each other, the inner sleeve is provided with a third pair of rollers constituted by the rollers, the two rollers in the third pair of rollers are both disposed between the two guide surfaces, and each roller is in rolling contact at least with one of the guide surfaces.

12. The telescopic cylinder according to claim 11, further comprising second adjustment assemblies corresponding to the guide assemblies, wherein the second adjustment assemblies each comprise a third adjusting member and a second adjusting rod;
the third adjusting member is configured to be oscillatingly mounted to the inner sleeve, and the third pair of rollers is rotatably mounted to the third adjusting member and is capable of oscillating with the third adjusting member; and
the second adjusting rod is connected to the third adjusting member, and the third adjusting member can be caused to oscillate by operating the second adjusting rod.

13. The telescopic cylinder according to claim 12, wherein
the third adjusting member comprises a third substrate and a third protrusion, the third pair of rollers is rotatably mounted to the third substrate and the third substrate is oscillatingly mounted to the inner sleeve, and the third protrusion protrudes from the third substrate; and
the second adjustment assembly further comprises a sleeve protrusion provided on the inner sleeve, the second adjusting rod extends in a direction perpendicular to the axial direction of the inner sleeve, and the second adjusting rod is inserted through the sleeve protrusion and threadingly engages with the third protrusion.

14. An X-ray imaging system, **characterized by** comprising the telescopic cylinder according to any one of claims 1 to 13.

15. The X-ray imaging system according to claim 14, further comprising a main body frame, an angle adjustment mechanism, and an X-ray generation mechanism,
the outermost sleeve of the telescopic cylinder is mounted to the main body frame in a manner such that the outermost sleeve is rotatable about the central axis thereof, and
the innermost sleeve of the telescopic cylinder is connected to the X-ray generation mechanism via the angle adjustment mechanism, and the X-ray generation mechanism is configured to be capable of being positioned on a front side of the telescopic cylinder.
